## Europäisches Patentamt
## European Patent Office
## Office européen des brevets

(19)

(11) Veröffentlichungsnummer: **0 307 498 B1**

(12)

# EUROPÄISCHE PATENTSCHRIFT

(45) Veröffentlichungstag der Patentschrift: 24.07.91

(51) Int. Cl.⁵: **A61F 6/14**

(21) Anmeldenummer: 87113573.7

(22) Anmeldetag: 16.09.87

(54) Empfängnisverhütende Intrauterinvorrichtung.

(43) Veröffentlichungstag der Anmeldung:
22.03.89 Patentblatt 89/12

(45) Bekanntmachung des Hinweises auf die
Patenterteilung:
24.07.91 Patentblatt 91/30

(84) Benannte Vertragsstaaten:
CH DE FR GB IT LI

(56) Entgegenhaltungen:
DE-A- 3 209 290
US-A- 3 786 808

(73) Patentinhaber: NAUCHNO-
PROIZVODSTVENNOE OBIEDINENIE
"MEDINSTRUMENT"
Ulitsa K. Tinchurina, 31
Kazan(SU)

(72) Erfinder: Vladimirovna Gainutdinova, Raisa
ulitsa Spartakovskaya, 80, kv. 15
Kazan(SU)
Erfinder: Indreevich Jurov, Boris
ulitsa Kurchatova, 6, kv. 99
Kazan(SU)
Erfinder: Moiseevich Mazo, Bentsian
ulitsa Gagarina, 73, kv. 51
Kazan(SU)
Erfinder: Mitrofanovna Petrova (Pokrovskaya),
Vera
ulitsa Frunze, 13a, kv.2
Kazan(SU)

(74) Vertreter: Finck, Dieter et al
Patentanwälte v. Füner, Ebbinghaus, Finck
Mariahilfplatz 2 & 3
W-8000 München 90(DE)

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 307 498 B1

## Beschreibung

Die Erfindung betrifft eine empfängnisverhütende Intrauterinvorrichtung mit einem Steg, mit einem mit dem Steg verbundenen, mit ihm ein T-förmiges Element bildenden Stab, mit einer auf dem Stab angebrachten Wendel und mit einem Faden zur Feststellung der Anwesenheit der empfängnisverhütenden Intrauterinvorrichtung innerhalb der Gebärmutterhöhle.

Aus der US-A-4 198 966 ist bereits eine empfängnisverhütende Intrauterinvorrichtung bekannt, die einen Steg, einen mit dem Steg verbundenen, mit ihm ein T-förmiges Element bildenden Stab, eine auf den Stab gewickelte Kupferwendel und einen am Stabende festeingeklemmten Faden aufweist, mit dem das Vorhandensein der Vorrichtung in der Gebärmutterhöhle dauernd überwacht werden kann.

Bekannt ist ferner eine empfängnisverhütende Intrauterinvorrichtung (Nova T Cu 200 Ag, Aktiengesellschaft Khukhtamyaki, Leiras, Finnland), die ebenfalls einen Steg, einen damit zu einem T-förmigen Element verbundenen Stab, eine auf dem Stab aufgebrachte Wendel aus Kupferdraht mit Silberunterlage und einen am Ende des Stabes festgelegten Faden aufweist, mit dem das Vorhandensein der empfängnisverhütenden Intrauterinvorrichtung innerhalb der Gebärmutterhöhle kontrolliert wird.

Der am Ende des Stabes durch einen Doppelknoten befestigte und sich durch den Gebärmutterhalskanal erstreckende Faden kann eine Reizung und als Folge davon eine Kontraktion der Muskulatur der Gebärmutter und eine Expulsion der ganzen Intrauterinvorrichtung verursachen.

Der Erfindung liegt die Aufgabe zugrunde, die empfängnisverhütende Intrauterinvorrichtung der gattungsgemäßen Art so auszubilden, daß eine Expulsion der Intrauterinvorrichtung während ihres Gebrauchs vermieden wird.

Diese Aufgabe wird erfindungsgemäß dadurch gelöst, daß der Faden über den Steg an dessen Verbindungsstelle mit dem Stab gelegt und längs des Stabs zwischen Stab und Wendel hindurchgezogen ist.

Zweckmäßigerweise ist die Intrauterinvorrichtung mit einer der Verbindungsstelle von Steg und Stab gegenüberliegenden Rille für die Aufnahme des Fadens versehen.

Bei der erfindungsgemäßen Ausgestaltung der empfängnisverhütenden Intrauterinvorrichtung entfällt ein Reizungen herbeiführender Knoten zur Befestigung des Fadens am Stab, so daß eine Expulsion nicht zu befürchten ist. Der Faden wird in der Regel aus Polyamid bzw. Polypropylen hergestellt. Dadurch wird eine Verletzungsgefahr im Bereich des inneren Muttermundes ausgeschlossen.

Anhand einer Zeichnung wird ein Ausführungsbeispiel der Erfindung näher erläutert. Es zeigt:

Fig. 1 die empfängnisverhütende Intrauterinvorrichtung in einer Gesamtansicht und

Fig. 2 die Intrauterinvorrichtung innerhalb der Gebärmutterhöhle.

Die in der Zeichnung gezeigte empfängnisverhütende Intrauterinvorrichtung hat einen Steg 1, mit dem ein Stab 2 unter Bildung eines T-förmigen Elements verbunden ist. Auf dem Stab 2 ist eine Wendel 3 angeordnet. An der Verbindungsstelle von Steg 1 und Stab 2 ist über den Steg 1 ein Faden 4 in einer im Steg 2 vorgesehenen Rille 5 gelegt und zwischen Stab 2 und Wendel 3 längs des Stabes 2 hindurchgezogen, wobei sein freies Ende aus dem Gebärmutterhalskanal 6 heraushängt.

Die empfängnisverhütende Intrauterinvorrichtung wird wie folgt gebraucht.

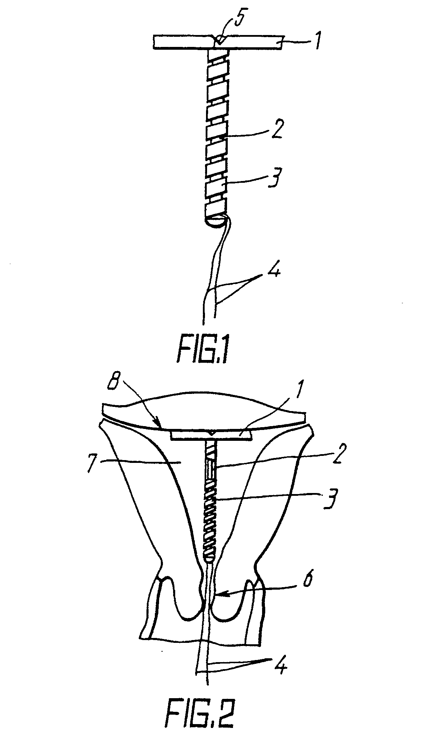

Die Intrauterinvorrichtung wird in eine nicht gezeigte Sonde eingesetzt, in der der Steg 1 und der Stab 2 mit der Wendel 3 und dem Faden 4 koaxial auf einer Linie zusammengelegt sind. Die empfängnisverhütende Intrauterinvorrichtung wird mit der Sonde unmittelbar in die Gebärmutterhöhle 7 (Fig. 2) eingeführt und in die der Gebärmutterform entsprechende Stellungslage gebracht. In der Gebärmutterhöhle 7 drückt die Intrauterinvorrichtung mit ihrem Steg 1 an dem Gebärmuttergrund 8, während der Faden 4 mit seinem freien Ende aus dem Gebärmutterhalskanal 6 heraushängt.

Durch die gewählte Erstreckung des Fadens 4 wird die Intrauterinvorrichtung atraumatisch und die Gefahr einer Expulsion herabgesetzt.

## Patentansprüche

1. Empfängnisverhütende Intrauterinvorrichtung mit einem Steg (1), mit einem mit dem Steg (1) verbundenen, mit ihm ein T-förmiges Element bildenden Stab (2), mit einer auf dem Stab (2) angebrachten Wendel (3) und mit einem Faden (4) zur Feststellung der Anwesenheit der empfängnisverhütenden Intrauterinvorrichtung innerhalb der Gebärmutterhöhle (7), dadurch **gekennzeichnet**, daß der Faden (4) über den Steg (1) an dessen Verbindungsstelle mit dem Stab (2) gelegt und längs des Stabes (2) zwischen Stab (2) und Wendel (3) durchgezogen ist.

2. Empfängnisverhütende Intrauterinvorrichtung nach Anspruch 1, dadurch **gekennzeichnet**, daß der Steg (1) auf seiner der Verbindungsstelle mit dem Stab (2) gegenüberliegenden Seite eine Rille (5) zur Aufnahme des Fadens (4) aufweist.

## Claims

1. A contraceptive intrauterine device with a crosspiece (1), with a bar (2) connected to the crosspiece (1) and forming a T-shaped element with the latter, with a helix (3) mounted on the bar (2) and with a thread (4) for ascertaining the presence of the contraceptive intrauterine device within the uterine cavity (7), characterized in that the thread (4) is laid over the crosspiece (1), at the junction point between the latter and the bar (2), and is drawn through along the bar (2), between the bar (2) and the helix (3).

2. A contraceptive intrauterine device in accordance with Claim 1, characterized in that on the opposite side to the junction point with the bar (2), the crosspiece (1) has a groove (5) to receive the thread (4).

## Revendications

1. Dispositif contraceptif intra-utérin avec une traverse (1), avec une branche (2) formant avec elle un élément en forme de T, avec une hélice (3) prévue sur la branche (2) et avec un fil (4) pour constater la présence du dispositif contraceptif intra-utérin à l'intérieur de la cavité utérine (7), caractérisé en ce que le fil (4) passe sur la traverse (1) à son point de jonction avec la branche (2) et est tiré le long de la branche (2) entre la branche (2) et l'hélice (3).

2. Dispositif contraceptif intra-utérin selon la revendication 1, caractérisé en ce que la traverse (1) présente, sur son côté opposé à son point de jonction avec la branche (2), une rainure (5) pour la réception du fil (4).

FIG.1

FIG.2